(19) Europäisches Patentamt
European Patent Office
Office européen des brevets

(11) Veröffentlichungsnummer: **0 259 750**
**A2**

(12) # EUROPÄISCHE PATENTANMELDUNG

(21) Anmeldenummer: 87112715.5

(51) Int. Cl.4: C07C 121/80 , A23K 1/16

(22) Anmeldetag: 01.09.87

(30) Priorität: 12.09.86 DE 3631009

(43) Veröffentlichungstag der Anmeldung: 16.03.88 Patentblatt 88/11

(84) Benannte Vertragsstaaten: AT BE CH DE ES FR GB GR IT LI NL SE

(71) Anmelder: BAYER AG
Konzernverwaltung RP Patentabteilung
D-5090 Leverkusen 1 Bayerwerk(DE)

(72) Erfinder: Lindel, Hans, Dr.
Carl-Duisberg-Strasse 321
D-5090 Leverkusen 1(DE)
Erfinder: Hallenbach, Werner, Dr.
Kleiststrasse 10
D-4018 Langenfeld(DE)
Erfinder: Berschauer, Friedrich, Dr.
Claudiusweg 9
D-5600 Wuppertal 1(DE)
Erfinder: De Jong, Anno, Dr.
Stockmannsmühle 46
D-5600 Wuppertal 1(DE)
Erfinder: Scheer, Martin, Dr.
Herberts-Katernberg 7
D-5600 Wuppertal 1(DE)

(54) **Arylethanolamine, Verfahren zu ihrer Herstellung und ihre Verwendung zur Leistungsförderung.**

(57) Die vorliegende Erfindung betrifft Arylethanolamine der Formel

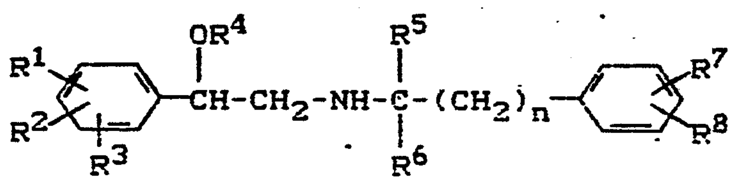

(I)

in welcher
$R^1$ für Cyano steht,
$R^2$ für Amino, Alkylamino oder Acylamino steht,
$R^3$ für Wasserstoff, Halogen, Alkyl, Halogenalkyl, Alkoxy, Halogenalkoxy, Hydroxymethyl, Nitro oder Methylsulfonylmethyl steht,
$R^4$ für Wasserstoff, Alkyl oder Acyl steht,
$R^5$ für Wasserstoff oder Alkyl steht,
$R^6$ für Alkyl steht,
n für 1, 2 oder 3 steht,
$R^7$ für die Reste $-COR^9$, $-O$-Alkylen-$COR^9$, -Alkylen-$R^{10}$ oder -O-Alkylen-$R^{10}$ steht,
$R^8$ für Wasserstoff, Alkyl oder Halogen steht,
$R^9$ für Hydroxy, Alkoxy oder den Rest $-NR^{11}R^{12}$ steht,
$R^{10}$ für Hydroxy, Alkoxy oder den Rest $-NR^{11}R^{12}$ steht,

EP 0 259 750 A2

$R^{11}$ für Wasserstoff oder Alkyl steht,

$R^{12}$ für Wasserstoff oder Alkyl steht,

sowie ihre physiologisch verträglichen Salze, Verfahren zu ihrer Herstellung und ihre Verwendung als Leistungsförderer für Tiere.

## Arylethanolamine, Verfahren zu ihrer Herstellung und ihre Verwendung zur Leistungsförderung

Die vorliegende Erfindung betrifft neue Arylethanolamine, Verfahren zu ihrer Herstellung und ihre Verwendung als Leistungsförderer für Tiere.

Arylethanolamine sind bekannte Verbindungen. Sie besitzen in Abhängigkeit von ihrer chemischen Struktur unterschiedliche pharmakologische Eigenschaften. Unter anderem besitzen bestimmte Arylethanolamine Wirkungen auf die Gewichtszunahme von Tieren sowie auf das Verhältnis der Bildung von Fleisch und Fett (EP-OS 26 298). Auch dabei scheint die Grundstruktur des Arylethanolamins von entscheidender Bedeutung für die Wirkung zu sein.

Es wurden gefunden:

1 die neuen Arylethanolamine der Formel I

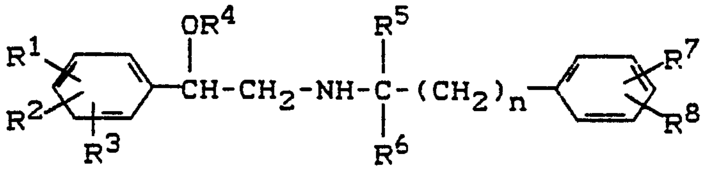

(I)

in welcher

$R^1$ für Cyano steht,

$R^2$ für Amino, Alkylamino oder Acylamino steht,

$R^3$ für Wasserstoff, Halogen, Alkyl, Halogenalkyl, Alkoxy, Halogenalkoxy, Hydroxymethyl, Nitro oder Methylsulfonylmethyl steht,

$R^4$ für Wasserstoff, Alkyl oder Acyl steht,

$R^5$ für Wasserstoff oder Alkyl steht,

$R^6$ für Alkyl steht,

n für 1, 2 oder 3 steht,

$R^7$ für die Reste $-COR^9$, $-O-Alkylen-COR^9$, $-Alkylen-R^{10}$ oder $-O-Alkylen-R^{10}$ steht,

$R^8$ für Wasserstoff, Alkyl oder Halogen steht,

$R^9$ für Hydroxy, Alkoxy oder den Rest $-NR^{11}R^{12}$ steht,

$R^{10}$ für Hydroxy, Alkoxy oder den Rest $-NR^{11}R^{12}$ steht,

$R^{11}$ für Wasserstoff oder Alkyl steht,

$R^{12}$ für Wasserstoff oder Alkyl steht,

sowie ihre physiologisch verträglichen Salze.

2. Verfahren zur Herstellung der Verbindungen der Formel I

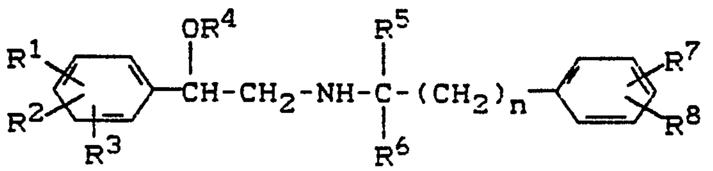

(I)

in welcher

$R^1$ für Cyano steht,

$R^2$ für Amino, Alkylamino oder Acylamino steht,

$R^3$ für Wasserstoff, Halogen, Alkyl, Halogenalkyl, Alkoxy, Halogenalkoxy, Hydroxymethyl, Nitro oder Methylsulfonylmethyl steht,

$R^4$ für Wasserstoff, Alkyl oder Acyl steht,

$R^5$ für Wasserstoff oder Alkyl steht,

$R^6$ für Alkyl steht,

n für 1, 2 oder 3 steht,

$R^7$ für die Reste $-COR^9$, $-O-Alkylen-COR^9$, $-Alkylen-R^{10}$ oder $-O-Alkylen-R^{10}$ steht,

$R^8$ für Wasserstoff, Alkyl oder Halogen steht,

$R^9$ für Hydroxy, Alkoxy oder den Rest $-NR^{11}R^{12}$ steht,

$R^{10}$ für Hydroxy, Alkoxy oder den Rest $-NR^{11}R^{12}$ steht,

$R^{11}$ für Wasserstoff oder Alkyl steht,
$R^{12}$ für Wasserstoff oder Alkyl steht,
dadurch gekennzeichnet,

a) daß man Halogenmethylketone der Formel II

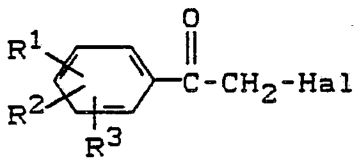

(II)

in welcher
$R^1$, $R^2$, $R^3$ die oben angegebene Bedeutung haben und Hal für Halogen steht,
mit Aminen der Formel III

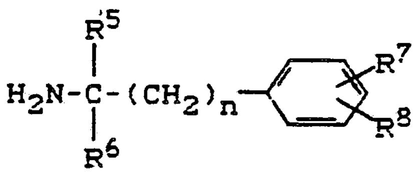

(III)

in welcher $R^5$ bis $R^8$ und n die oben angegebene Bedeutung haben,
umsetzt und anschließend die Carbonylgruppe reduziert, oder

b) daß man Epoxide der Formel IV

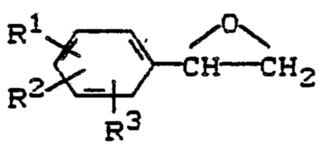

(IV)

in welcher $R^1$, $R^2$, $R^3$ die oben angegebene Bedeutung haben,
mit Aminen der Formel III

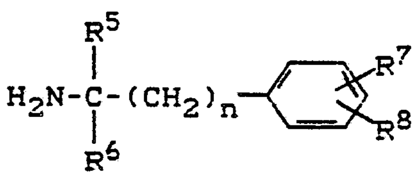

(III)

in welcher $R^5$ bis $R^8$ und n die oben angegebene Bedeutung haben,
umsetzt, oder

c) daß man $\beta$-Halogenethylverbindungen der Formel V

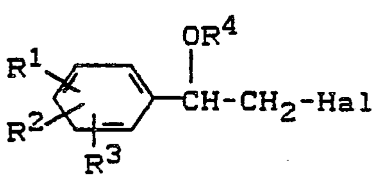

(V)

in welcher
$R^1$ bis $R^4$ die oben angegebene Bedeutung haben und Hal für Halogen steht,
mit Aminen der Formel III

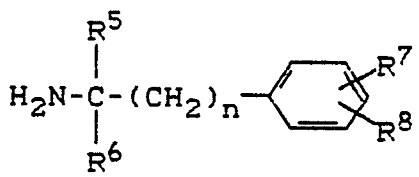

(III)

in welcher $R^5$ bis $R^8$ und n die oben angegebene Bedeutung haben, umsetzt oder

d) für den Fall, daß in Formel I $R^5$ für Wasserstoff steht, daß man Verbindungen der Formel VI

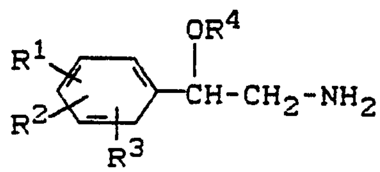

(VI)

in welcher
$R^1$ bis $R^4$ die oben angegebene Bedeutung haben, mit Verbindungen der Formel VII

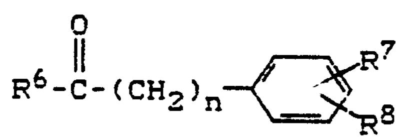

(VII)

in welcher $R^6$, $R^7$, $R^8$ und n die oben angegebene Bedeutung haben,
unter reduzierenden Bedingungen umsetzt, oder

e) daß man Verbindungen der Formel VIII

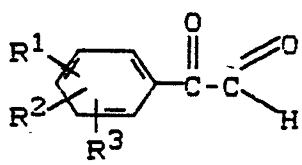

(VIII)

in welcher $R^1$, $R^2$, $R^3$ die oben angegebene Bedeutung haben, mit Aminen der Formel III

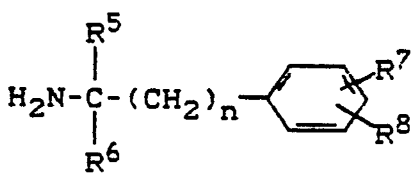

(III)

in welcher $R^5$ bis $R^8$ und n die oben angegebene Bedeutung haben,
unter reduzierenden Bedingungen umsetzt.

Die Verbindungen der Formel I können auch in Form ihrer Racemate sowie als Gemische der zueinander diastereomeren oder enantiomeren Formen vorliegen.

Physiologisch verträgliche Salze der Verbindungen der Formel I können mit folgenden Säuren gebildet werden:

Salzsäure, Schwefelsäure, Phosphorsäure, Perchlorsäure, Brom-, Iodwasserstoffsäure, Salpetersäure, Essigsäure, Oxalsäure, Malonsäure, Bernsteinsäure, Ascorbinsäure, Äpfelsäure, Weinsäure, Maleinsäure, Fumarsäure, Methansulfonsäure, Benzoesäure, substituierte Benzoesäuren, Ameisensäure, Toluolsulfonsäure, Benzolsulfonsäure, Phthalsäure, Naphthalinsulfonsäure, Nikotinsäure, Palmitinsäure, Embonsäure.

Bevorzugt sind Verbindungen der Formel I, in welcher
$R^1$ für Cyano steht,
$R^2$ für Amino, $C_1$-$C_3$-Alkylamino oder $C_1$-$C_3$-Acylamino steht,
$R^3$ für Wasserstoff, Halogen, Halogenalkyl oder Nitro steht,
$R^4$ für Wasserstoff, Methyl, Acetyl oder Benzoyl steht,
$R^5$ für Wasserstoff oder $C_1$-$C_3$-Alkyl steht,
$R^6$ für $C_1$-$C_3$-Alkyl steht,
n für 1,2 oder 3 steht,
$R^7$ für die Reste $-COR^9$, -O-Alkylen-$COR^9$, -Alkylen-$R^{10}$ oder -O-Alkylen-$R^{10}$ steht,
$R^8$ für Wasserstoff, Halogen oder $C_1$-$C_3$-Alkyl steht,
$R^9$ für Hydroxy, Alkoxy oder den Rest $NR^{11}R^{12}$ steht,
$R^{10}$ für Hydroxy, Alkoxy oder den Rest $NR^{11}R^{12}$ steht,
$R^{11}$ für Wasserstoff oder $C_1$-$C_3$-Alkyl steht,
$R^{12}$ für Wasserstoff oder $C_1$-$C_3$-Alkyl steht.

Besonders bevorzugt sind Verbindungen der Formel I, in welcher
$R^1$ für Cyano steht,
$R^2$ für Amino, Methylamino oder Acetylamino steht,
$R^3$ für Wasserstoff, Fluor, Chlor, Brom oder Trifluormethyl steht,
$R^4$ für Wasserstoff oder Acetyl steht,
$R^5$ für Wasserstoff steht,
$R^6$ für Methyl oder Ethyl steht,
n für 1 oder 2 steht,
$R^7$ für die Reste $-COR^9$, $-O-CH_2-COR^9$, $-CH_2-R^{10}$ oder $-O-CH_2-CH_2-R^{10}$ steht,
$R^8$ für Wasserstoff, Chlor oder Methyl steht,
$R^9$ für Hydroxy, $C_1$-$C_3$-Alkoxy oder den Rest $NR^{11}R^{12}$ steht,
$R^{10}$ für Hydroxy, Methoxy oder den Rest $NR^{11}R^{12}$ steht,
$R^{11}$ für Wasserstoff oder Methyl steht,
$R^{12}$ für Wasserstoff oder Methyl steht.

Ganz besonders bevorzugt sind Verbindungen der Formel I, in welcher
$R^1$ für Cyano steht,
$R^2$ für Amino steht,
$R^3$ für Wasserstoff oder Chlor steht,
$R^4$ für Wasserstoff steht,
$R^5$ für Wasserstoff steht,
$R^6$ für Methyl steht,
n für 1 steht
$R^7$ für die Reste $-COR^9$, $-OCH_2COR^9$ oder $-OCH_2CH_2R^{10}$ steht,
$R^8$ für Wasserstoff steht,
$R^9$ für Hydroxy oder Methoxy steht,
$R^{10}$ für Hydroxy oder den Rest $NR^{11}R^{12}$ steht,
$R^{11}$ für Wasserstoff steht,
$R^{12}$ für Wasserstoff oder Methyl steht.

Im einzelnen seien neben den Beispielen die folgenden Verbindungen der Formel I genannt:

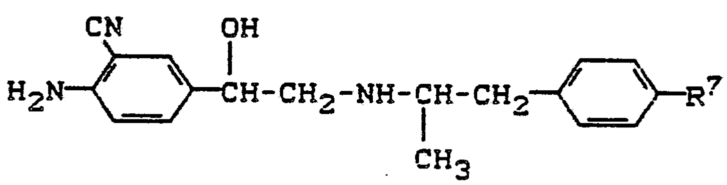

$R^7$
$OCH_2COOH$
$COOH$
$OCH_2CH_2NH_2$
$OCH_2CH_2NHCH_3$

Bevorzugt seien die Salze mit Salzsäure, Schwefelsäure, Phosphorsäure, Oxalsäure, Maleinsäure, Fumarsäure, Malonsäure genannt.

Die neuen Verbindungen der Formel I lassen sich nach den oben angegebenen Verfahren 2a) bis 2e) herstellen.

Setzt man bei Verfahren 2a) als Halogenmethylketon der Formel II (3-Cyano-4-amino-5-chlorphenyl)-brommethylketon und als Amin der Formel III 3-(4-Carbomethoxyphenyl)-2-propylamin ein, läßt sich Verfahren 2a) durch folgendes Reaktionsschema wiedergeben:

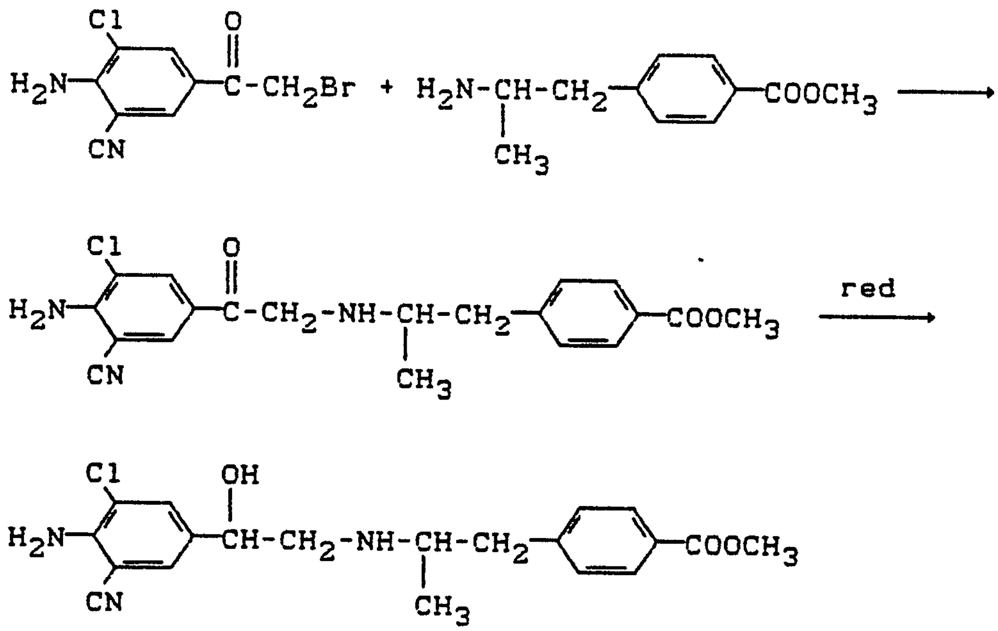


Die Verbindungen der Formel II sind bekannt (vgl. z.B. US-Pat. 4.404.222). Die Substituenten $R^1$, $R^2$ und $R^3$ besitzen bevorzugt die weiter oben bei den Verbindungen der Formel I angegebenen bevorzugten Bedeutungen. Im einzelnen seien folgende Verbindungen der Formel II genannt:

(3-Cyano-4-aminophenyl)-brommethylketon,
(3-Cyano-4-aminophenyl)-chlormethylketon,
(3-Cyano-4-amino-5-chlorphenyl)-brommethylketon,
(3-Cyano-4-amino-5-trifluormethylphenyl)-brommethylketon,
(3-Cyano-4-acetaminophenyl)-chlormethylketon,
(3-Cyano-4-methylamino-5-bromphenyl)-brommethylketon.

Die Amine der Formel III sind bekannt (vgl. z.B. EP-OS 23 385). Die Substituenten $R^5$, $R^6$, $R^7$, $R^8$ und n besitzen bevorzugt die weiter oben bei den Verbindungen der Formel I angegebenen bevorzugten Bedeutungen. Im einzelnen seien folgende Verbindungen der Formel III genannt:

3-(4-Carbomethoxyphenyl)-2-propylamin,
3-(4-Methoxycarbonylmethoxyphenyl)-2-propylamin,
3-(4-Carboxyphenyl)-2-propylamin,
3-(4-Carboxymethoxyphenyl)-2-propylamin,
3-(4-Hydroxymethylphenyl)-2-propylamin,
3-(4-Dimethylaminomethylphenyl)-2-propylamin,
3-(4-(2-Hydroxyethyl)phenyl)-2-propylamin.

Als Reduktionsmittel zur Durchführung des Verfahrens 2a) seien folgende Reduktionsmittel genannt: $H_2$/Katalysator, als Katalysator seien beispielsweise genannt: $PtO_2$, Pd-Aktivkohle;
komplexe Metallhydride wie z.B. $LiAlH_4$, $NaBH_4$, $NaBH_3CN$.

Besonders bevorzugt werden folgende Reduktionsmittel eingesetzt:
$NaBH_4$ und $NaBH_3CN$.

Verfahren 2a) wird durchgeführt indem man die Verbindungen II und III in einem Verdünnungsmittel in etwa äquimolarem Verhältnis zusammengibt und anschließend reduziert.

Die Reduktion wird bei Temperaturen von -20°C bis +100°C bevorzugt durchgeführt.

Es wird bevorzugt bei Normaldruck gearbeitet.

Als Verdünnungsmittel dienen alle inerten organischen Lösungsmittel. Hierzu gehören insbesondere aliphatische und aromatische Kohlenwasserstoffe wie Pentan, Hexan, Cyclohexan, Petrolether, Ligroin, Benzol, Toluol; chlorierte Kohlenwasserstoffe wie Methylenchlorid, Ethylenchlorid, Chloroform; Ether wie Diethylether und Glykoldimethylether; Nitrile wie Acetonitril, Propionitril und Benzonitril; Alkohole wie Methanol, Ethanol, n-und i-Propanol.

Bevorzugt sind Alkohole, wobei ohne Isolierung der Zwischenstufen sofort die Reduktion durchgeführt werden kann.

Setzt man bei Verfahren 2b) als Epoxid der Formel IV (3-Cyano-4-amino-5-chlorphenyl)ethylenoxid und als Amin der Formel III 3-(4-Dimethylaminomethylphenyl)-2-propylamin ein, läßt sich Verfahren 2b) durch folgendes Reaktionsschema wiedergeben:

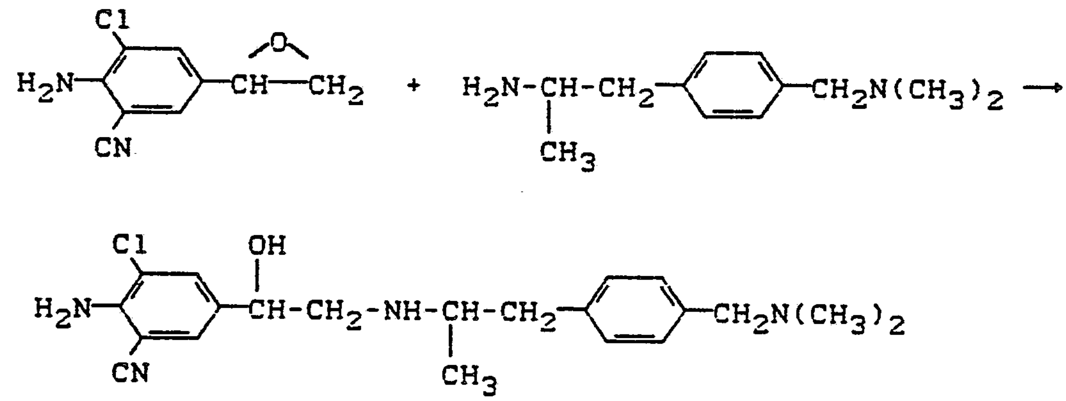

Epoxide der Formel V sind bekannt (vgl. z.B. US-Pat. 4 404 222). Die Substituenten $R^1$, $R^2$, $R^3$ besitzen bevorzugt die weiter oben bei den Verbindungen der Formel I angegebenen bevorzugten Bedeutungen.

Im einzelnen seien folgende Verbindungen der Formel IV genannt:

(3-Cyano-4-aminophenyl)-ethylenoxid,

(3-Cyano-4-amino-5-chlorphenyl)-ethylenoxid,

(3-Cyano-4-amino-5-trifluormethylphenyl)-ethylenoxid,

(3-Cyano-4-methylaminophenyl)-ethylenoxid.

Verfahren 2b) wird durchgeführt, indem man etwa äquimolare Mengen des Epoxids der Formel IV und des Amins der Formel III in einem Verdünnungsmittel umsetzt.

Im allgemeinen wird ein Überschuß an Amin (1-3 molar, bevorzugt 1-1,5 molar) bezogen auf das Epoxid der Formel IV verwendet.

Die Reaktion wird bei Temperaturen von +20 bis +150°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, ferner Nitrile wie Acetonitril und Benzonitril, Amide wie Dimethylformamid, Alkohole wie Methanol, Ethanol, n-und i-Propanol.

Bevorzugt sind Alkohole.

Setzt man bei Verfahren 2c) als β-Halogenethylverbindung der Formel V 1-(3-Cyano-4-aminophenyl)-1-hydroxy-2-chlorethan und als Amin der Formel III 3-(4-Carbomethoxyphenyl)-2-propylamin ein, läßt sich Verfahren 2c) durch folgendes Schema wiedergeben:

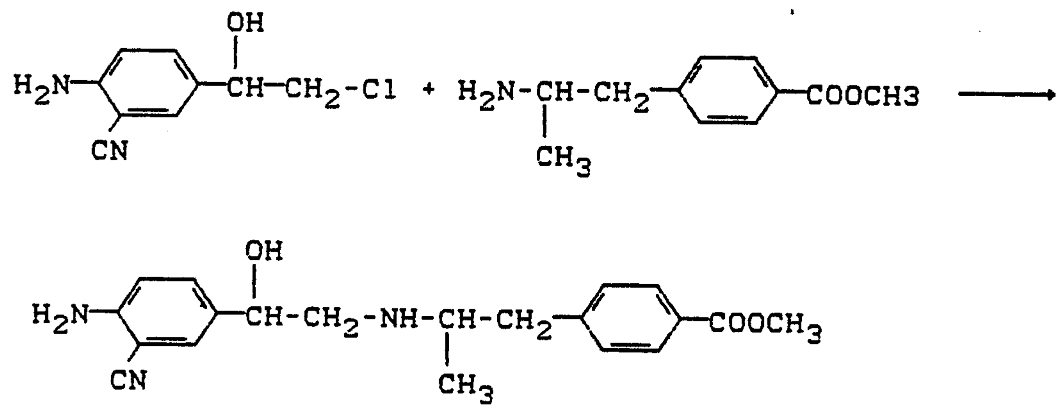

β-Halogenethylverbindungen der Formel V sind bekannt (vgl. z.B. US-Pat. 4 404 222). Die Substituenten $R^1$ bis $R^4$ besitzen bevorzugt die weiter oben bei den Verbindungen der Formel I genannten bevorzugten Bedeutungen. Im einzelnen seien folgende Verbindungen der Formel V genannt:
1-(3-Cyano-4-aminophenyl)-2-chlorethanol,
1-(3-Cyano-4-aminophenyl)-2-bromethanol,
1-(3-Cyano-4-amino-5-chlorphenyl)-2-chlorethanol,
1-(3-Cyano-4-amino-5-nitrophenyl)-2-chlorethanol,
1-(3-Cyano-4-amino-5-nitrophenyl)-2-bromethanol,
1-(3-Cyano-4-amino-5-trifluormethylphenyl)-2-chlorethanol.

Verfahren 2c) wird durchgeführt indem man die β-Halogenethylverbindung der Formel V mit überschüssigem Amin der Formel III gegebenenfalls in Gegenwart eines Verdünnungsmittels umsetzt.

Die Reaktion wird bei Temperaturen von +20 bis +150°C durchgeführt.

Es wird bei Normaldruck oder unter erhöhtem Druck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Cyclohexan, Benzol, Toluol, Methylenchlorid, Chloroform, ferner Ether wie Diethylether, Tetrahydrofuran und Dioxan, weiterhin Nitrile wie Acetonitril und Benzonitril, ferner Amide wie Dimethylformamid, ferner Alkohole wie Methanol, Ethanol, n-und i-Propanol.

Bevorzugt eingesetzt werden Alkohole.

Setzt man bei Verfahren 2d) als Verbindung der Formel VI 1-(3-Cyano-4-amino-5-chlorphenyl)-2-aminoethanol und als Verbindung der Formel VII 4-(Dimethylaminomethyl)phenylaceton ein, läßt sich Verfahren 2d) durch folgendes Reaktionsschema wiedergeben:

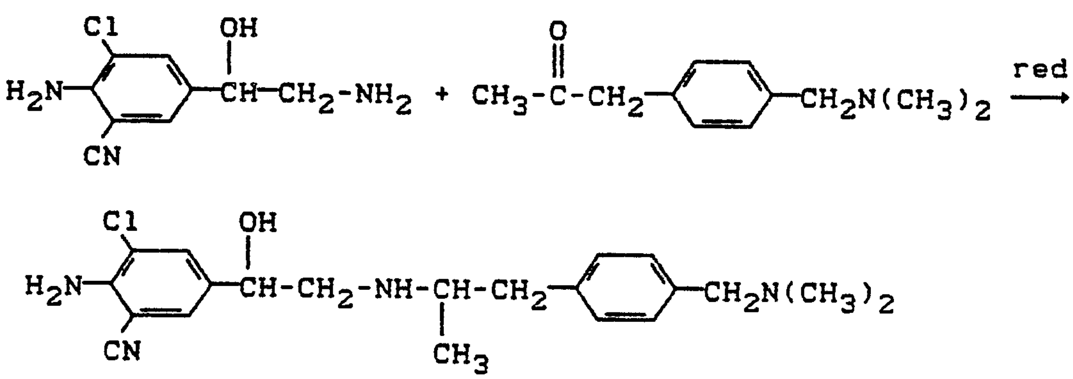


Verbindungen der Formel VI sind bekannt (vgl. z.B. US-Pat. 4 404 222). Die Substituenten $R^1$ bis $R^4$ besitzen bevorzugt die weiter oben bei den Verbindungen der Formel I angegebenen bevorzugten Bedeutungen. Im einzelnen seien folgende Verbindungen der Formel VI genannt:
1-(3-Cayno-4-aminophenyl)-2-aminoethanol,
1-(3-Cyano-4-amino-5-bromphenyl)-2-aminoethanol,
1-(3-Cyano-4-methylaminophenyl)-2-aminoethanol,
1-(3-Cyano-4-amino-5-trifluormethylphenyl)-2-aminoethanol.

Verbindungen der Formel VII sind bekannt (vgl. z.B. EP-OS 70 133). Die Substituenten $R^6$ bis $R^8$ und n besitzen bevorzugt die weiter oben bei den Verbindungen der Formel I angegebenen bevorzugten Bedeutungen.

Im einzelnen seien folgende Verbindungen der Formel VII genannt:
4-Carbomethoxyphenylaceton,
4-(Carbomethoxymethoxy)phenylaceton
4-(2-Hydroxyethoxy)phenylaceton.

Verfahren 2d) wird durchgeführt, indem man etwa äquimolare Mengen der Verbindungen der Formel VI und VII in einem Verdünnungsmittel vorlegt und die Mischung reduziert.

Die Reaktion wird bei Temperaturen von 0°C bis 150°C durchgeführt.

Es wird bevorzugt bei Normaldruck gearbeitet.

Als Verdünnungsmittel dienen alle inerten organischen Lösungsmittel. Hierzu gehören aliphatische und aromatische, gegebenenfalls halogenierte Kohlenwasserstoffe wie Pentan, Hexan, Cyclohexan, Benzol, Toluol, Methylenchlorid, Ethylenchlorid, Chloroform, Chlorbenzol, ferner Ether wie Diethylether, Tetrahydrofuran, Dioxan, weiterhin Nitrile wie Acetonitril, Benzonitril, Amide wie Dimethylformamid, Alkohole wie Methanol, Ethanol.

Als Reduktionsmittel dienen:
$H_2$/Katalysator, als Katalysator sei beispielsweise $PtO_2$ genannt; komplexe Metallhydride wie z.B. $LiAlH_4$, $NaBH_4$, $NaBH_3CN$.

Setzt man bei Verfahren 2e) als Verbindung der Formel VIII 3-Cyano-4-aminophenylglyoxal und als Amin der Formel III 3-(4-Methoxyphenyl)-2-propylamin ein, läßt sich Verfahren 2e) durch folgendes Reaktionsschema wiedergeben:

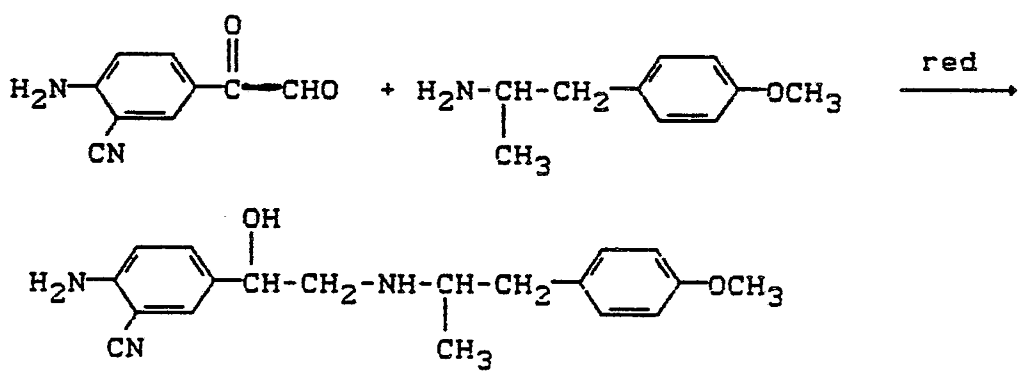


Verbindungen der Formel VIII sind bekant (vgl. z.B. US-Pat. 4 404 222). Die Substituenten $R^1$, $R^2$, $R^3$ besitzen bevorzugt die weiter oben bei den Verbindungen der Formel I genannten bevorzugten Bedeutungen. Im einzelnen seien folgende Verbindungen der Formel VIII genannt:
3-Cyano-4-aminophenylglyoxal,
3-Cyano-4-amino-5-chlorphenylglyoxal,
3-Cyano-4-amino-5-bromphenylglyoxal,
3-Cyano-4-amino-5-trifluormethylphenylglyoxal.

Verfahren 2e) wird durchgeführt, indem man zur Verbindung der Formel VIII in einem Verdünnungsmittel etwa die äquivalente Menge des Amins der Formel III zusetzt und anschließend reduziert.

Die Reaktion wird bei Temperaturen von 0°C bis 100°C durchgeführt.

Es wird bevorzugt bei Normaldruck gearbeitet.

Als Verdünnungsmittel dienen alle inerten organischen Lösungsmittel. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl-und Dibutylether, Glykoldimethylether und Diglykoldimethylether, Tetrahydrofuran und Dioxan, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Tetramethylensulfon und Hexamethylphosphorsäuretriamid, weiterhin Alkohole wie Methanol, Ethanol, n-und i-Propanol.

Als Reduktionsmittel dienen
$H_2$/Katalysator; als Katalysator seien $PtO_2$ und Pd-Kohle genannt, ferner komplexe Metallhydride wie $LiAlH_4$ und $NaBH_4$.

Die Wirkstoffe werden als Leistungsförderer bei Tieren zur Förderung und Beschleunigung des Wachstums, der Milch-und Wollproduktion, sowie zur Verbesserung der Futterverwertung, der Fleischqualität und zur Verschiebung des Fleisch-Fett-Verhältnisses zugunsten von Fleisch eingesetzt. Die Wirkstoffe werden bei Nutz-, Zucht-, Zier-und Hobbytieren verwendet.

Zu den Nutz-und Zuchttieren zählen Säugetiere wie z.B. Rinder, Schweine, Pferde, Schafe, Ziegen, Kaninchen, Hasen, Damwild; Pelztiere wie Nerze, Chinchilla; Geflügel wie z.B. Hühner, Gänse, Enten, Truthähne, Tauben; Fische wie z.B. Karpfen, Forellen, Lachse, Aale, Schleien, Hechte; Reptilien wie z.B. Schlangen und Krokodile.

Zu den Zier-und Hobbytieren zählen Säugetiere wie Hunde und Katzen; Vögel wie Papageien, Kanarienvögel; Fische wie Zier-und Aquarienfische, z.B. Goldfische.

Die Wirkstoffe werden unabhängig vom Geschlecht der Tiere während allen Wachstums-und Leistungsphasen der Tiere eingesetzt. Bevorzugt werden die Wirkstoffe während der intensiven Wachstums-und Leistungsphase eingesetzt. Die intensive Wachstums-und Leistungsphase dauert je nach Tierart von einem Monat bis zu 10 Jahren.

Die Menge der Wirkstoffe, die den Tieren zur Erreichung des gewünschten Effektes verabreicht wird, kann wegen der günstigen Eigenschaften der Wirkstoffe weitgehend variiert werden. Sie liegt vorzugsweise bei etwa 0,001 bis 50 mg/kg, insbesondere 0,01 bis 5 mg/kg Körpergewicht pro Tag. Die passende Menge des Wirkstoffs sowie die passende Dauer der Verabreichung hängen insbesondere von der Art, dem Alter, dem Geschlecht, der Wachstums-und Leistungsphase, dem Gesundheitszustand und der Art der Haltung und Fütterung der Tiere ab und sind durch jeden Fachmann leicht zu ermitteln.

Die Wirkstoffe werden den Tieren nach den üblichen Methoden verabreicht. Die Art der Verabreichung hängt insbesondere von der Art, dem Verhalten und dem Gesundheitszustand der Tiere ab.

Die Wirkstoffe können einmalig verabreicht werden. Die Wirkstoffe können aber auch während der ganzen oder während eines Teils der Wachstums-und Leistungsphase temporär oder kontinuierlich verabreicht werden.

Bei kontinuierlicher Verabreichung kann die Anwendung ein-oder mehrmals täglich in regelmäßigen oder unregelmäßigen Abständen erfolgen.

Die Verabreichung erfolgt oral oder parenteral in dafür geeigneten Formulierungen oder in reiner Form.

Die Wirkstoffe können in den Formulierungen allein oder in Mischung mit anderen leistungsfördernden Wirkstoffen, mineralischen Futtermitteln, Spurenelement-Verbindungen, Vitaminen, Nicht-Protein-Verbindungen, Farbstoffen, Antioxidantien, Aromastoffen, Emulgatoren, Fließhilfsstoffen, Konservierungs-und Preßhilfsstoffen vorliegen.

Andere leistungsfördernde Wirkstoffe sind:
z.B. Antibiotika wie Tylosin und Virginamycin. Mineralische Futtermittel sind z.B. Dicalciumphosphat, Magnesiumoxid, Natriumchlorid.
Spurenelement-Verbindungen sind z.B. Eisenfumarat, Natriumjodid, Kobaltchlorid, Kupfersulfat, Zinkoxid. Vitamine sind z.B. Vitamin A, Vitamin $D_3$, Vitamin E, B-Vitamine, Vitamin C.

Nicht-Protein-Verbindungen sind z.B. Biuret, Harnstoff. Farbstoffe sind z.B. Carotinoide wie Citranaxanthin, Zeaxanthin, Capsanthin. Antioxidantien sind z.b. Ethoxyquin, Butylhydroxy-Toluol.
Aromastoffe sind z.B. Vanillin. Emulgatoren sind z.B. Ester der Milchsäure, Lecithin. Fließhilfsstoffe sind z.B. Natriumstearat, Calciumstearat.

Konservierungsstoffe sind z.B. Zitronensäure, Propionsäure.
Preßhilfsstoffe sind z.B. Ligninsulfonate, Celluloseether.

Die Verabreichung der Wirkstoffe kann auch zusammen mit dem Futter und/oder dem Trinkwasser erfolgen.

Zum Futter zählen Einzelfuttermittel pflanzlicher Herkunft wie Heu, Rüben, Getreidenebenprodukte, Einzelfuttermittel tierischer Herkunft wie Fleisch, Fette, Milchprodukte, Knochenmehl, Fischprodukte, die Einzelfuttermittel wie Vitamine, Proteine, Aminosäuren z.b. DL-Methionin, Salze wie Kalk und Kochsalz. Zum Futter zählen auch Ergänzungs-, Fertig-und Mischfuttermittel. Diese enthalten Einzelfuttermittel in einer Zusammensetzung, die eine ausgewogene Ernährung hinsichtlich der Energie-und Proteinversorgung sowie der Versorgung mit Vitaminen, Mineralsalzen und Spurenelementen sicherstellt.

Die Konzentration der Wirkstoffe im Futter beträgt normalerweise etwa 0,01-500 ppm, bevorzugt 0,1-50 ppm.

Die Wirkstoffe können als solche oder in Form von Prämixen oder Futterkonzentraten dem Futter zugesetzt werden.

Beispiel für die Zusammensetzung eines Kükenaufzuchtfutters, das erfindungsgemäßen Wirkstoff enthält:

200 g Weizen, 340 g Mais, 361 g Sojaschrot, 60 g Rindertalg, 15 g Dicalciumphosphat, 10 g Calciumcarbonat, 4 g jodiertes Kochsalz, 7,5 g Vitamin-Mineral-Mischung und 2,5 g Wirkstoff-Prämix ergeben nach sorgfältigem Mischen 1 kg Futter.

In einem kg Futtermischung sind enthalten: 600 I.E. Vitamin A, 100 I.E. Vitamin $D_3$, 10 mg Vitamin E, 1 mg Vitamin $K_3$, 3 mg Riboflavin, 2 mg Pyridoxin, 20 mcg Vitamin $B_{12}$, 5 mg Calciumpantothenat, 30 mg Nikotinsäure, 200 mg Cholinchlorid, 200 mg $MnSO_4 \times H_2O$, 140 mg $ZnSO_4 \times 7\ H_2O$, 100 mg $FeSO_4 \times 7\ H_2O$ und 20 mg $CuSO_4 \times 5\ H_2O$.

2,5 g Wirkstoff-Prämix enthalten z.B. 10 mg Wirkstoff, 1 g DL-Methion, Rest Sojabohnenmehl.

Beispiel für die Zusammensetzung eines Schweineaufzuchtfutters, das erfindungsgemäßen Wirkstoff enthält:

630 g Futtergetreideschrot (zusammengesetzt aus 200 g Mais, 150 g Gerste-, 150 g Hafer-und 130 g Weizenschrot), 80 g Fischmehl, 60 g Sojaschrot, 60 g Tapiokamehl, 38 g Bierhefe, 50 g Vitamin-Mineral-Mischung für Schweine, 30 g Leinkuchenmehl, 30 g Maiskleberfutter, 10 g Sojaöl, 10 g Zuckerrohrmelasse und 2 g Wirkstoff-Prämix (Zusammensetzung z.B. wie beim Kükenfutter) ergeben nach sorgfältigem Mischen 1 kg Futter.

Die angegebenen Futtergemische sind zur Aufzucht und Mast von vorzugsweise Küken bzw. Schweinen abgestimmt, sie können jedoch in gleicher oder ähnlicher Zusammensetzung auch zur Fütterung anderer Tiere verwendet werden.

## Beispiel A

Ratten-Fütterungsversuch

Weibliche Laborratten 90-110 g schwer vom Typ SPF Wistar (Züchtung Hagemann) werden ad lib mit Standard Rattenfutter, das mit der gewünschten Menge Wirkstoff versetzt ist, gefüttert. Jeder Versuchsansatz wird mit Futter der identischen Charge durchgeführt, so daß Unterschiede in der Zusammensetzung des Futters die Vergleichbarkeit der Ergebnisse nicht beeinträchtigen können.

Die Ratten erhalten Wasser ad lib.

Jeweils 12 Ratten bilden eine Versuchsgruppe und werden mit Futter, das mit der gewünschten Menge Wirkstoff versetzt ist, gefüttert. Eine Kontrollgruppe erhält Futter ohne Wirkstoff. Das durchschnittliche Körpergewicht sowie die Streuung in den Körpergewichten der Ratten ist in jeder Versuchsgruppe gleich, so daß eine Vergleichbarkeit der Versuchsgruppen untereinander gewährleistet ist.

Während des 13-tägigen Versuchs werden Gewichtszunahme und Futterverbrauch bestimmt und die relative Gewichtszunahme im Vergleich zur unbehandelten Kontrolle errechnet.

Es werden die aus der Tabelle ersichtlichen Ergebnisse erhalten:

### Ratten-Fütterungsversuch

| Wirkstoff (Bsp.-Nr.) | Dosis 25 ppm relat. Gewichtszunahme (%) |
|---|---|
| 1 | 38 |
| 2 | 33 |
| 3 | 63 |
| 4 | 32 |
| 5 | 53 |

Beispiele

Allgemeine Vorschrift für Verfahren 2a

Herstellung der Verbindungen der Formel I nach Verfahren 2a

10 mmol der Verbindung der Formel II werden bei 0°C portionsweise zu einer Lösung von 10 mmol des Amins der Formel III in 15 ml absolutem Ethanol gegeben. Man läßt auf 10-15°C kommen und rührt bei dieser Temperatur eine Stunde nach. Dann wird wieder auf 0°C abgekühlt und portionsweise werden 600 mg (50 mmol) Natriumborhydrid zugegeben. Man rührt über Nacht bei Raumtemperatur nach. Nach Zugabe von 20 ml Wasser wird 30 min gerührt, eingedampft und zwischen Wasser und Essigester verteilt. Die organische Phase wird über Natriumsulfat getrocknet und eingedampft. Der Rückstand wird umkristallisiert.

Allgemeine Vorschrift für Verfahren 2b

Herstellung der Verbindungen der Formel I nach Verfahren 2b

0,1 mol der Verbindung der Formel IV und 0,11 mol des Amins der Formel III werden in 200 ml Methanol über Nacht unter Rückfluß erhitzt. Das Lösungsmittel und überschüssiges Amin werden abgezogen und der Rückstand umkristallisiert.

Allgemeine Vorschrift für Verfahren 2c

Herstellung der Verbindungen der Formel I nach Verfahren 2c

10 mmol der Verbindung der Formel V werden in 150 ml Ethanol gelöst und 20 ml des Amins der Formel III werden zugegeben, die Mischung wird 18 Stunden unter Rückfluß erhitzt. Dann werden Lösungsmittel und überschüssiges Amin abgezogen, und der Rückstand wird in 100 ml trockenem Ether aufgenommen. Das unlösliche Aminhydrohalogenid wird abfiltriert, die etherische Lösung mit Wasser gewaschen, über Natriumsulfat getrocknet und eingedampft. Das Rohprodukt wird umkristallisiert.

Allgemeine Vorschrift für Verfahren 2d

Herstellung der Verbindungen der Formel I nach Verfahren 2d

Zu 22 mmol der Verbindung VI in 10 ml absolutem Ethanol werden 22 mmol der Carbonylverbindung der Formel VII bei 0-5°C zugegeben. Man läßt auf Raumtemperatur kommen und rührt noch 30 Minuten nach. Die Lösung wird dann zu 0,15 g Adams-Katalysator (vorhydriert in 10 ml absolutem Ethanol) gegeben und die Mischung 4-5 Stunden bei 40°C und 50 atm Wasserstoffdruck hydriert. Nach Abfiltrieren vom Katalysator wird eingedampft und umkristallisiert.

Allgemeine Vorschrift für Verfahren 2e

Herstellung der Verbindungen der Formel I nach Verfahren 2e

Zur Lösung von 10 mmol der Verbindung der Formel VIII in 50 ml Ethanol werden bei 10-15°C 15 mmol des Amins der Formel III getropft. Man läßt auf Raumtemperatur kommen und rührt noch 15 min nach. Dann verdünnt man mit weiteren 100 ml Ethanol und gibt bei 0-5°C portionsweise 80 mmol Natriumborhydrid zu. Man läßt auf Raumtemperatur kommen und rührt über Nacht. Dann gibt man bei 10°C 200 ml Wasser zu, rührt 30 min., dampft das Ethanol ab und extrahiert den Rückstand dreimal mit je 40 ml Dichlormethan. Die vereinigten organischen Phasen werden mit 100 ml Wasser gewaschen, über Natriumsulfat getrocknet und eingedampft.

Nach den oben angegebenen Verfahren 2a) bis 2e) werden die folgenden Verbindungen hergestellt:

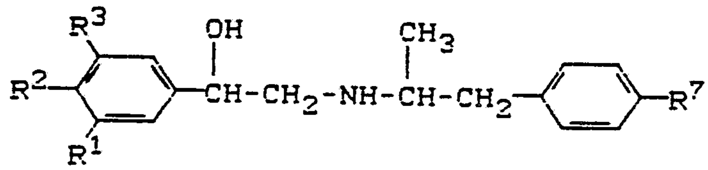

| Bsp.-Nr. | $R^1$ | $R^2$ | $R^3$ | $R^7$ | $^1H$-NMR (DMSO-$d_6$, δ [ppm]) |
|---|---|---|---|---|---|
| 1 | CN | $NH_2$ | H | $COOCH_3$ | 0,95 (d,3H); 2,5-2,9 (m,5H); 3,9 (s,3H); 4,4 (m,1H); 5,9 (s,2H); 6,7 (d,1H); 7,3 (m,4H); 7,9 (m,2H). |
| 2 | CN | $NH_2$ | H | $OCH_2CH_2OH$ | 0,9 (dd,3H); 2,4-2,9 (m, 5H); 3,7 (m,2H); 3,9 (m, 2H); 4,4 (m1H); 4,8 (s, 1H); 5,9 (s,2H); 6,8 (m, 3H); 7,0 (m, 3H); 7,2 (m,1H). |
| 3 | CN | $NH_2$ | H | $OCH_2COOCH_3$ | 0,9 (dd,3H); 2,4-2,8 (m, 5H); 3,8 (s,3H); 4,4 (s,2H); 4,5 (m,1H); 5,9 (s, 2H); 6,8 (m, 3H); 7,0-7,2 (m, 4H). |
| 4 | CN | $NH_2$ | Cl | $COOCH_3$ | 1,1 (d,3H); 2,4-3,0 (m, 5H); 3,9 (s,3H); 4,4 (m, 1H); 4,7 (s,2H); 7,2 (m, 3H); 7,9 (m,3H). |
| 5 | CN | $NH_2$ | Cl | $OCH_2COOCH_3$ | 1,1 (d,3H); 2,4-3,0 (m, 5H); 3,8 (s,3H); 4,5 (m, 1H); 4,6 (s,2H); 4,8 (s,2H); 6,9 (m, 3H); 7,1 m (3H) |

## Ansprüche

1. Arylethanolamine der Formel

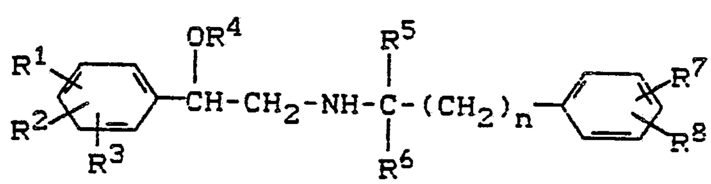

(I)

in welcher
$R^1$ für Cyano steht,
$R^2$ für Amino, Alkylamino oder Acylamino steht,

$R^3$ für Wasserstoff, Halogen, Alkyl, Halogenalkyl, Alkoxy, Halogenalkoxy, Hydroxymethyl, Nitro oder Methylsulfonylmethyl steht,
$R^4$ für Wasserstoff, Alkyl oder Acyl steht,
$R^5$ für Wasserstoff oder Alkyl steht,
$R^6$ für Alkyl steht,
n für 1, 2 oder 3 steht,
$R^7$ für die Reste $-COR^9$, $-O$-Alkylen-$COR^9$, -Alkylen-$R^{10}$ oder $-O$-Alkylen-$R^{10}$ steht,
$R^8$ für Wasserstoff, Alkyl oder Halogen steht,
$R^9$ für Hydroxy, Alkoxy oder den Rest $-NR^{11}R^{12}$ steht,
$R^{10}$ für Hydroxy, Alkoxy oder den Rest $-NR^{11}R^{12}$ steht,
$R^{11}$ für Wasserstoff oder Alkyl steht,
$R^{12}$ für Wasserstoff oder Alkyl steht,
sowie ihre physiologisch verträglichen Salze.

2. Verfahren zur Herstellung der Verbindungen der Formel I,

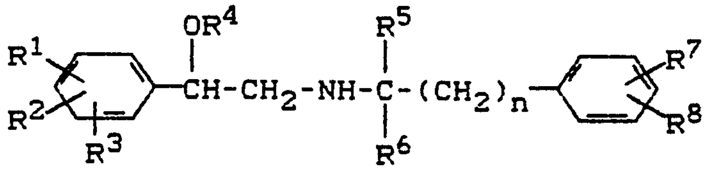

(I)

in welcher
$R^1$ für Cyano steht,
$R^2$ für Amino, Alkylamino oder Acylamino steht,
$R^3$ für Wasserstoff, Halogen, Alkyl, Halogenalkyl, Alkoxy, Halogenalkoxy, Hydroxymethyl, Nitro oder Methylsulfonylmethyl steht,
$R^4$ für Wasserstoff, Alkyl oder Acyl steht,
$R^5$ für Wasserstoff oder Alkyl steht,
$R^6$ für Alkyl steht,
n für 1, 2 oder 3 steht,
$R^7$ für die Reste $-COR^9$, $-O$-Alkylen-$COR^9$, -Alkylen-$R^{10}$ oder $-O$-Alkylen-$R^{10}$ steht,
$R^8$ für Wasserstoff, Alkyl oder Halogen steht,
$R^9$ für Hydroxy, Alkoxy oder den Rest $-NR^{11}R^{12}$ steht,
$R^{10}$ für Hydroxy, Alkoxy oder den Rest $-NR^{11}R^{12}$ steht,
$R^{11}$ für Wasserstoff oder Alkyl steht,
$R^{12}$ für Wasserstoff oder Alkyl steht,
dadurch gekennzeichnet,

a) daß man Halogenmethylketone der Formel II

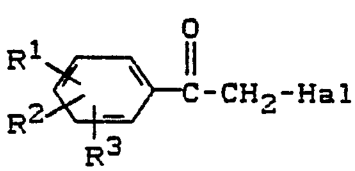

(II)

in welcher
$R^1$, $R^2$, $R^3$ die oben angegebene Bedeutung haben und Hal für Halogen steht,
mit Aminen der Formel III

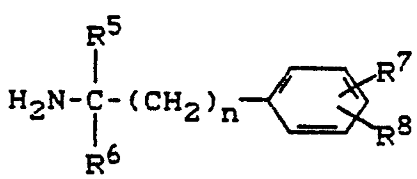

(III)

in welcher $R^5$ bis $R^8$ und n die oben angegebene Bedeutung haben, umsetzt und anschließend die Carbonylgruppe reduziert, oder

b) daß man Epoxide der Formel IV

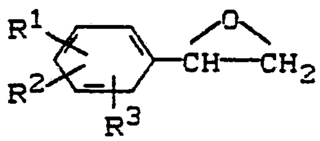

(IV)

in welcher $R^1$, $R^2$, $R^3$ die oben angegebene Bedeutung haben, mit Aminen der Formel III

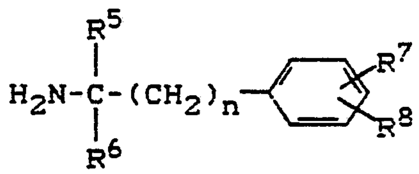

(III)

in welcher $R^5$ bis $R^8$ und n die oben angegebene Bedeutung haben, umsetzt, oder

c) daß man $\beta$-Halogenethylverbindungen der Formel V

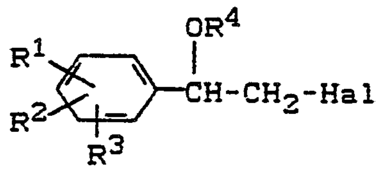

(V)

in welcher

$R^1$ bis $R^4$ die oben angegebene Bedeutung haben und Hal für Halogen steht, mit Aminen der Formel III

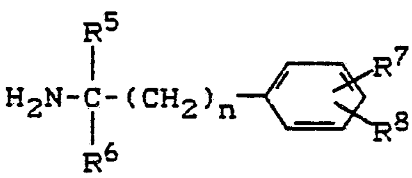

(III)

in welcher $R^5$ bis $R^8$ und n die oben angegebene Bedeutung haben, umsetzt oder

d) für den Fall, daß in Formel I $R^5$ für Wasserstoff steht, daß man Verbindungen der Formel VI

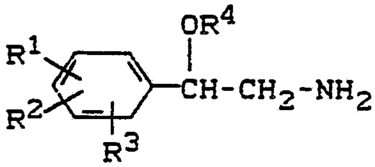

(VI)

in welcher

$R^1$ bis $R^4$ die oben angegebene Bedeutung haben, mit Verbindungen der Formel VII

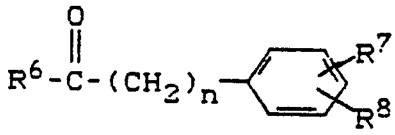

(VII)

in welcher $R^6$, $R^7$, $R^8$ und n die oben angegebene Bedeutung haben, unter reduzierenden Bedingungen umsetzt, oder

e) daß man Verbindungen der Formel VIII

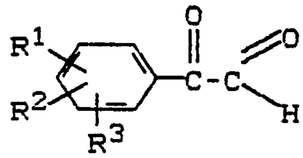

(VIII)

in welcher $R^1$, $R^2$, $R^3$ die oben angegebene Bedeutung haben, mit Aminen der Formel III

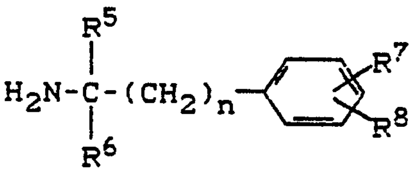

(III)

in welcher $R^5$ bis $R^8$ und n die oben angegebene Bedeutung haben, unter reduzierenden Bedingungen umsetzt.

3. Mittel zur Leistungsförderung von Tieren, gekennzeichnet durch einen Gehalt an Arylethanolaminen der Formel I gemäß Anspruch 1.

4. Tierfutter, Trinkwasser für Tiere, Zusätze für Tierfutter und Trinkwasser für Tiere, gekennzeichnet durch einen Gehalt an Arylethanolaminen der Formel I gemäß Anspruch 1.

5. Verwendung von Arylethanolaminen der Formel I gemäß Anspruch 1 zur Leistungsförderung von Tieren.

6. Verfahren zur Herstellung von Mitteln zur Leistungsförderung von Tieren, dadurch gekennzeichnet, daß man Arylethanolamine der Formel I gemäß Anspruch 1 mit Streck-und/oder Verdünnungsmitteln versetzt.

7. Verfahren zur Herstellung von Tierfutter, Trinkwasser für Tiere oder Zusätze für Tierfutter und Trinkwasser für Tiere, dadurch gekennzeichnet, daß man Arylethanolamine der Formel I gemäß Anspruch 1 mit Futtermitteln oder Trinkwasser und gegebenenfalls weiteren Hilfsstoffen vermischt.